(19) Europäisches Patentamt
European Patent Office
Office européen des brevets

(11) Veröffentlichungsnummer: **0 257 295**
**B1**

(12) # EUROPÄISCHE PATENTSCHRIFT

(45) Veröffentlichungstag der Patentschrift: **29.08.90**

(51) Int. Cl.[5]: **C07C 255/64**

(21) Anmeldenummer: **87110462.6**

(22) Anmeldetag: **20.07.87**

(54) **(Z)-2-Cyan-2-oximino-acetyl-chloride sowie ein Verfahren zu ihrer Herstellung.**

(30) Priorität: **28.07.86 DE 3625434**

(43) Veröffentlichungstag der Anmeldung: **02.03.88 Patentblatt 88/9**

(45) Bekanntmachung des Hinweises auf die Patenterteilung: **29.08.90 Patentblatt 90/35**

(84) Benannte Vertragsstaaten: **BE CH DE FR GB IT LI NL**

(56) Entgegenhaltungen:
**EP-A- 0 201 999**
**CH-A- 646 143**

(73) Patentinhaber: **BAYER AG, D-5090 Leverkusen 1 Bayerwerk(DE)**

(72) Erfinder: **Lunkenheimer, Winfried, Dr., Bismarckstrasse 29, D-5600 Wuppertal 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 257 295 B1

## Beschreibung

Die vorliegende Erfindung betrifft neue Z-Isomere von N-(2-Cyan-2-oximino)-acetylchlorid, ein Verfahren zu deren Herstellung und ihre Verwendung als Zwischenprodukte zur Synthese von E/Z-Isomerengemischen und reinen Z-Isomeren von 2-Cyan-2-oximino-acetamiden, welche als Pflanzenschutzmittel, besonders als Fungizide eingesetzt werden können.

Die neuen (Z)-2-Cyan-2-oximino-acetylchloride sind durch die allgemeine Formel (I)

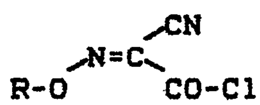

(I)

in welcher

R für gegebenenfalls ein- bis dreifach, gleich oder verschieden durch Halogen oder Methyl substituiertes Cycloalkyl mit 3 bis 6 Kohlenstoffatomen oder für die Gruppierung

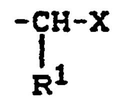

steht, wobei

$R^1$ für Wasserstoff oder Methyl steht und

X für Wasserstoff, für geradkettiges oder verzweigtes Alkyl mit 1 bis 8 Kohlenstoffatomen, für Vinyl, Ethinyl, Cyano, für gegebenenfalls ein- oder zweifach, gleich oder verschieden, durch Halogen substituiertes Cyclopropyl, für gegebenenfalls durch Halogen oder Alkyl mit 1 bis 4 Kohlenstoffatomen substituiertes Phenyl oder für einen gegebenenfalls substituierten und gegebenenfalls mit einem Benzolring annellierten Heterocyclus steht, welcher 1 bis 3 gleiche oder verschiedenen Heteroatome aus der Gruppe Stickstoff, Sauerstoff und Schwefel enthält und durch Halogen oder Alkyl mit 1 bis 4 Kohlenstoffatomen substituiert sein kann.

Weiterhin wurde gefunden, daß man die neuen (Z)-2-Cyan-2-oximino-acetylchloride der allgemeinen Formel (I) erhält, wenn man Verbindungen der allgemeinen Formel (II)

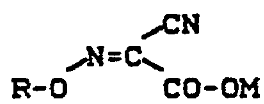

(II)

in welcher

R die oben angegebene Bedeutung hat und

M für Wasserstoff oder ein Alkalimetallatom wie beispielsweise Natrium oder Kalium steht, mit einem Halogenierungsmittel gegebenenfalls in Gegenwart eines Verdünnungsmittels und gegebenenfalls in Gegenwart eines Katalysators umsetzt.

Die neuen (Z)-2-Cyan-2-oximino-acetylchloride sind interessante Zwischenprodukte zur Herstellung von Pflanzenschutz-Wirkstoffen. So eignen sich die Stoffe der allgemeinen Formel (I) z.B. als Ausgangsstoffe zur Synthese von E/Z-Isomerengemischen und den reinen Z-Isomeren und E-Isomeren von $N^{\alpha}$-(2-Cyan-2-alkoximinoacetyl)-aminosäurederivaten und -peptiden, welche alle sehr gute fungizide Wirksamkeit besitzen.

Überraschenderweise sind die E/Z-Isomerengemische und die reinen Z-Isomeren und E-Isomeren von $N^{\alpha}$-(2-Cyan-2-alkoximinoacetyl)-aminosäurederivaten und -peptiden, die sich aus den erfindungsgemäßen (Z)-2-Cyan-2-oximino-acetylchloriden der allgemeinen Formel (I) durch Umsetzung mit den entsprechenden Aminen in Gegenwart eines Säurebindemittels herstellen lassen, der aus dem Stand der Technik bekannten Verbindung Zink-ethylen-1,2-bis-dithiocarbamat bezüglich ihrer fungiziden Wirksamkeit überlegen, EP-A 257 294

Die erfindungsgemäßen Stoffe sind durch die allgemeine Formel (I) allgemein definiert.

Besonders bevorzugt sind diejenigen Verbindungen der allgemeinen Formel (I), in denen

R für gegebenenfalls ein- oder zweifach, gleich oder verschieden durch Chlor oder Methyl substituiertes Cyclopentyl oder Cyclohexyl oder für die Gruppierung

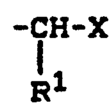

steht, wobei

$R^1$ für Wasserstoff oder Methyl steht und

X für Wasserstoff, Methyl, Ethyl, Vinyl, Ethinyl, Cyano, für gegebenenfalls ein- oder zweifach durch

Chlor substituiertes Cyclopropyl, für gegebenenfalls durch Chlor, Methyl oder Ethyl substituiertes Phenyl, für gegebenenfalls durch Chlor oder Methyl substituiertes Pyrazol-1-yl oder 1,2,4-Triazol-1-yl oder für den Rest

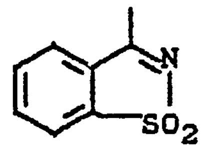

steht.

Verwendet man beispielsweise Kalium-(Z)-2-cyan-2-methoximino-acetat als Ausgangsstoff und Oxalylchlorid als Halogenierungsmittel, so kann der Reaktionsablauf des erfindungsgemäßen Verfahrens durch das folgende Formelschema wiedergegeben werden:

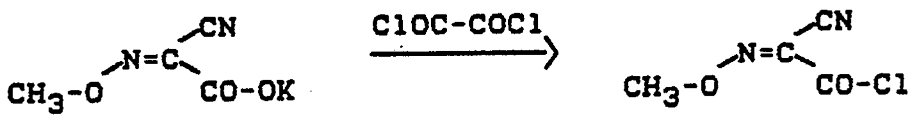


Die für die Durchführung des erfindungsgemäßen Verfahrens als Ausgangsstoffe benötigten Verbindungen sind durch die allgemeine Formel (II) allgemein definiert. In dieser Formel steht R vorzugsweise für diejenigen Bedeutungen, die bereits im Zusammenhang mit der Beschreibung der erfindungsgemäßen Stoffe der allgemeinen Formel (I) für den Rest R aufgeführt wurden. M steht vorzugsweise für Wasserstoff, Kalium oder Natrium, insbesondere für Natrium oder Kalium.

Die Verbindungen der allgemeinen Formel (II) sind bisher noch nicht bekannt. Sie lassen sich jedoch nach im Prinzip bekannten Verfahren in einfacher Weise herstellen. So erhält man beispielsweise die Verbindungen der allgemeinen Formel (II), indem man die Carbonsäureester der allgemeinen Formel (III)

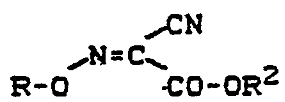

in welcher
R die oben angegebene Bedeutung hat und
$R^2$ für Methyl oder Ethyl steht,
in einem Lösungsmittel, wie beispielsweise Wasser, Methanol oder Ethanol mit einem Alkalihydroxid, wie beispielsweise Kalium- oder Natriumhydroxid verseift oder zunächst mit einem Alkalihydroxid, wie beispielsweise Natriumhydroxid, und anschließend mit einer Säure, wie beispielsweise Salzsäure oder Schwefelsäure, oder mit einem sauren Ionenaustauscher in Gegenwart eines Verdünnungsmittels, wie beispielsweise Wasser, Alkohole, Ether oder Gemische von Alkoholen oder Ethern mit Wasser, bei Temperaturen zwischen 0 und 80 °C, vorzugsweise zwischen 20 und 40 °C, umsetzt.

Die Carbonsäureester der allgemeinen Formel (III) sind teilweise bekannt (vgl. z.B. J. Antibiot. 37, 557 (1984)).

Sie können erhalten werden, indem man Amide der allgemeinen Formel (IV)

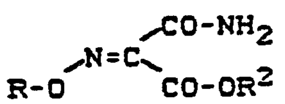 (IV)

in welcher
R und $R^2$ die oben angegebene Bedeutung haben,
mit einem Dehydratisierungsmittel, wie beispielsweise Trifluoressigsäureanhydrid, Trichloracetylchlorid, Mesylchlorid, Tosylchlorid, Titantetrachlorid oder N,N-Dimethylchlorformimidiumchlorid, in Gegenwart einer tertiären Base wie beispielsweise Pyridin, Triethylamin oder N-Methylmorpholin, und gegebenenfalls in Gegenwart eines Lösungsmittels, wie beispielsweise Dioxan, Tetrahydrofuran, Acetonitril, Methylenchlorid oder Pyridin bei Temperaturen zwischen -20 und 60 °C dehydratisiert.

Die Amide der allgemeinen Formel (IV) sind teilweise bekannt (vgl. z.B. J. Antibiot, 37, 557 (1984)).

Sie können erhalten werden, indem man Verbindungen der allgemeinen Formel (V)

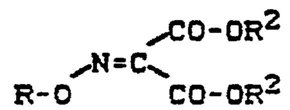

(V)

in welcher
R und $R^2$ die oben angegebenen Bedeutungen haben, mit Ammoniak in Gegenwart eines Lösungsmittels, wie beispielsweise Methanol, Ethanol, Diethylether, Dimethoxyethan, Dimethylformamid oder Acetonitril bei Temperaturen zwischen -20 °C und 30 °C umsetzt.

Die Verbindungen der allgemeinen Formel (V) sind teilweise bekannt (vgl. z.B. J. Antibiot. 37, 557 (1984)).

Sie können erhalten werden, indem man Verbindungen der allgemeinen Formel (VI)

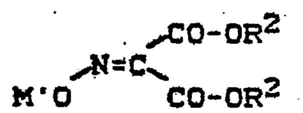

(VI)

in welcher
$R^2$ die oben angegebene Bedeutung hat und
M' für Wasserstoff oder für ein Alkalimetallatom, wie beispielsweise Natrium oder Kalium steht,
mit Verbindungen der allgemeinen Formel (VII)
R-W (VII)
in welcher
R die oben angegebene Bedeutung hat und
W für Halogen oder einen Sulfonyloxy-Rest, wie vorzugsweise Chlor, Brom, Jod, $-OSO_2OCH_3$, $-OSO_2OC_2H_5$, $-OSO_2CH_3$ oder

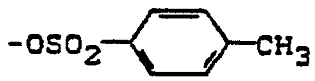

steht,
gegebenenfalls in Gegenwart einer Base, wie beispielsweise Kaliumcarbonat, Triethylamin oder Diazabicycloundecan (DBU), und in Gegenwart eines Lösungsmittels, wie beispielsweise Aceton, Dimethylformamid, Dimethylsulfoxid oder Acetonitril, bei Temperaturen zwischen 0 und 120 °C umsetzt.

Die Verbindungen der allgemeinen Formeln (VI) und (VII) sind allgemein bekannte Verbindungen der organischen Chemie.

Als Verdünnungsmittel kommen für das erfindungsgemäße Verfahren inerte organische Lösungsmittel in Frage. Hierzu gehören vorzugsweise Ether, wie Diethylether; chlorierte Kohlenwasserstoffe, wie Methylenchlorid, Ester, wie Essigester; Nitrile, wie Acetonitril; sowie Kohlenwasserstoffe, wie Toluol.

Das erfindungsgemäße Verfahren wird mit einem Halogenierungsmittel durchgeführt. Man kann alle üblicherweise verwendbaren Halogenierungsmittel einsetzen. Vorzugsweise arbeitet man mit Oxalylchlorid, Phosphorpentachlorid, Thionylchlorid oder Phosgen. Dabei ist darauf zu achten, daß die Halogenierungsmittel keinen freien Chlorwasserstoff enthalten, da sonst während des Verfahrens Isomerisierung zum E-Isomer erfolgen kann.

Das erfindungsgemäße Verfahren wird gegebenenfalls in Gegenwart eines Katalysators durchgeführt. Hierzu gehören vorzugsweise Dimethylformamid und Triphenylphosphin.

Die Reaktionstemperaturen können bei der Durchführung des erfindungsgemäßen Verfahrens in einem größeren Bereich variiert werden. Im allgemeinen arbeitet man bei Temperaturen zwischen -20 °C und 80 °C, vorzugsweise bei Temperaturen zwischen -10 °C und 30 °C, insbesondere bei 0 °C.

Bei der Durchführung des erfindungsgemäßen Verfahrens setzt man auf 1 Mol der Verbindung der allgemeinen Formel (II) im allgemeinen 1 bis 10 Mol, vorzugsweise 1 bis 3 Mol Halogenierungsmittel ein. Zur Isolierung der Verbindung der allgemeinen Formel (I) wird das Reaktionsgemisch in üblicher Art und Weise aufgearbeitet.

Die erfindungsgemäßen (Z)-2-Cyan-2-oximino-acetylchloride der allgemeinen Formel (I) eignen sich als Zwischenprodukte zur Synthese von E/Z-Isomerengemischen und den reinen Z- und E-Isomeren von 2-Cyan-2-oximino-acetamiden, welche als Pflanzenschutzmittel, vorzugsweise als Fungizide Verwendung finden können.

So lassen sich beispielsweise 2-Cyan-2-oximino-acetamide der allgemeinen Formel (VIII)

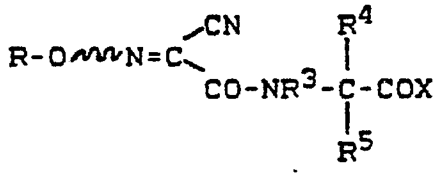

(VIII)

in welcher
R für Alkyl steht,
$R^3$ für Wasserstoff, Alkyl, für gegebenenfalls substituiertes Phenyl sowie für gegebenenfalls substituiertes Benzyl;
$R^4$ für Wasserstoff oder Alkyl steht;
$R^5$ für Wasserstoff, Alkyl, Alkoxycarbonylalkyl, Hydroxycarbonylalkyl, Aminocarbonylalkyl, Azolylalkyl, Cyanalkyl, Hydroxyalkyl, Alkenyl, Alkinyl, gegebenenfalls substituiertes Cycloalkyl, für gegebenenfalls substituiertes Phenyl und Phenylalkyl sowie für die Gruppierung $R^6$-$SO_n$-Z- steht, wobei
$R^6$ für Wasserstoff, Alkyl sowie gegebenenfalls substituiertes Phenylalkyl steht;
n für die Zahlen 0, 1 oder 2 steht und
Z für eine geradkettige oder verzweigte Alkylenkette steht;
$R^3$ und $R^5$ gemeinsam mit dem Stickstoff-Atom und dem Kohlenstoffatom, an die sie gebunden sind, für einen 5- oder 6-gliedrigen Heterocyclus stehen;
$R^4$ und $R^5$ gemeinsam mit dem Kohlenstoffatom, an das sie gebunden sind, für Cycloalkyliden stehen und
X für die Gruppierung -$OR^I$ oder $NR^{II}R^{III}$ steht, wobei
$R^I$ für Wasserstoff, Alkyl, Alkenyl oder für Alkinyl steht;
$R^{II}$ für Wasserstoff oder Alkyl steht ;
$R^{III}$ für Wasserstoff, Alkyl, Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl, gegebenenfalls substituiertes Cycloalkyl steht
oder
$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann,
herstellen, indem man die (Z)-2-Cyan-2-oximinoacetylchloride der allgemeinen Formel (I)

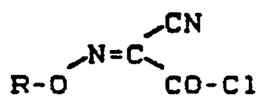

(I)

in welcher
R für Alkyl steht
mit Aminosäure-Derivaten der allgemeinen Formel (IX)

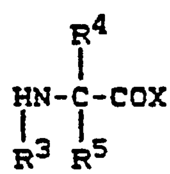

(IX)

in welcher
$R^3$, $R^4$, $R^5$ und X die oben angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt.

Als Verdünnungsmittel kommen für die Umsetzung der erfindungsgemäßen (Z)-2-Cyan-2-oximinoacetylchloride der allgemeinen Formel (I) mit Aminosäure-Derivaten der allgemeinen Formel (IX) inerte organische Lösungsmittel in Frage. Hierzu gehören Ketone, wie Aceton oder Ethylmethylketon; Ester wie Ethyl-oder Methylacetat; Amide wie Dimethylformamid; Nitrile wie Acetonitril. Chlorkohlenwasserstoffe, wie Methylenchlorid oder Chloroform, Kohlenwasserstoffe, wie Toluol; oder Ether, wie Tetrahydrofuran; bzw. deren Mischungen.

Als Säurebindemittel kommen für die Umsetzung der erfindungsgemäßen (Z)-2-Cyan-2-oximino-acetylchloride der allgemeinen Formel (I) mit Aminosäure-Derivaten der allgemeinen Formel (IX) übliche anorganische und organische Säurebinder in Frage. Hierzu gehören vorzugsweise tertiäre Amine, wie Triethylamin, Py ridin oder N-Methylmorpholin; sowie anorganische Basen, wie Natriumcarbonat oder Calciumcarbonat.

Die Umsetzung der erfindungsgemäßen (Z)-2-Cyan-2-oximino-acetylchloride der allgemeinen Formel (I) mit Aminosäure-Derivaten der allgemeinen Formel (IX) wird gegebenenfalls in Gegenwart eines Katalysators durchgeführt. Beispielsweise genannt seien 4-Dimethylaminopyridin, 1-Hydroxybenzotriazol oder Dimethylformamid.

Die Temperaturen können bei der Umsetzung der erfindungsgemäßen (Z)-2-Cyan-2-oximino-acetylchloride der allgemeinen Formel (I) mit Aminosäure-Derivaten der allgemeinen Formel (IX) in einem größeren Bereich variiert werden. Im allgemeinen arbeitet man zwischen -60 bis +120 °C,vorzugsweise bei -20 bis +40 °C.

Die Verbindungen der allgemeinen Formel (VIII) fallen je nach Reaktionsbedingungen als E/Z-Isomerengemische oder als reine Z-Isomere an. Dabei wird die E/Z-Isomerisierung durch die Anwesenheit von freier Chlorwasserstoffsäure begünstigt.

Bei der Umsetzung der erfindingsgemäßen (Z)-2-Cyan-2-oximino-acetylchloride der allgemeinen Formel (I) mit Aminosäure-Derivaten der allgemeinen Formel (IX) arbeitet man vorzugsweise in äquimolaren Mengen, wobei die Base je nach gewünschtem Reaktionsprodukt (E/Z-Isomerengemisch oder reines Z-Isomeres) im Unter- oder Überschuß eingesetzt wird.

Die aus den erfindungsgemäßen Verbindungen der allgemeinen Formel (I) herstellbaren E/Z-Isomerengemische und reinen Z-Isomere von N$^{\alpha}$-(2-Cyan-2-alkoximinoacetyl)-aminosäurederivaten der allgemeinen Formel (VIII) besitzen sehr gute fungizide Eigenschaften.

Herstellungsbeispiele

Beispiel 1

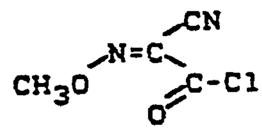

Man suspendiert 23,5 g (0,14 Mol) gut getrocknetes Kalium-(Z)-2-cyan-2-methoximino-acetat in 350 ml absolutem Ether, gibt einige Tropfen absolutes Dimethylformamid hinzu, tropft bei 0 °C in 35 Minuten 53,4 g (0,42 Mol) Oxalylchlorid (zuvor mit 2 g Kaliumcarbonat 1 Stunde verrührt) zu und rührt 2 Stunden bei 0 °C. Das Reaktionsgemisch wird filtriert, das Filtrat im Vakuum eingedampft und der Rückstand zweimal mit trockenem Dichlormethan im Vakuum abgedampft.

Man erhält 15,7 g (76 % der Theorie) (Z)-2-Cyan-2-methoximino-acetylchlorid als orangefarbenes Öl, das sofort weiter umgesetzt wird.

Herstellung des Ausgangsproduktes:

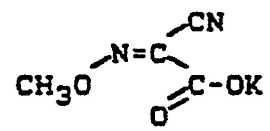

In eine Lösung von 34,3 g (0,2 Mol) (Z)-2-Cyan-2-methoximino-essigsäure-ethylester (91prozentig) in 200 ml Ethanol tropft man eine Lösung von 11,8 g (0,21 Mol) Kaliumhydroxid in 80 ml Wasser und rührt 1,5 Stunden bei 40 °C. Die Reaktionslösung wird im Vakuum bei 40 °C eingedampft, der Rückstand mit Ethanol und Ether gewaschen und im Vakuum bei Raumtemperatur getrocknet.

Man erhält 28,3 g (85 % der Theorie) Kalium-(Z)-2-Cyan-2-methoximino-acetat als beigefarbenes Pulver, das sich bei 111 °C (Peak-Temperatur der Differentialthermoanalyse) explosionsartig zersetzt.
Die Z-Konfiguration ist durch $^{13}$C-NMR sichergestellt.

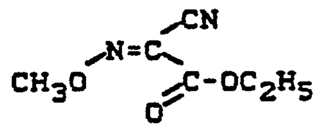

In eine Lösung von 33,5 g (0,177 Mol) 92prozentigem (Z)-2-Methoximino-malonsäure-monoamid-ethylester und 28,0 g (0,35 Mol) Pyridin in 260 ml trockenem Dioxan tropft man bei 0 °C in 25 Minuten 71,4 g (0,336 Mol) Trifluoressigsäureanhydrid (99prozentig) und rührt 2 Stunden bei Raumtemperatur. Das Reaktionsgemisch wird mit 260 ml Dichlormethan verdünnt, zweimal mit je 200 ml Wasser, mit 150 ml 10prozentiger Natriumhydrogencarbonatlösung und 200 ml Wasser gewaschen, über Natriumsulfat getrocknet und im Vakuum eingedampft.

Man erhält in einer Reinheit von 88 % (GC) 23,4 g (74 % der Theorie) (Z)-2-cyan-2-methoximinoessigsäureethylester als rotbraunes Öl vom Brechungsindex $n_D^{20}$ = 1,4399.

Die Z-Konfiguration ist durch $^{13}$C-NMR sichergestellt.

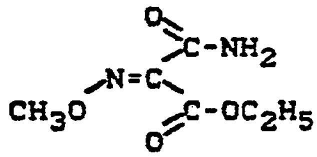

In eine Lösung von 44,2 g (0,196 Mol) 90prozentigem 2-Methoximino-malonsäurediethylester in 200 ml absolutem Ethanol tropft man bei Raumtemperatur 115 ml (0,587 Mol) einer 5,1 M Lösung von Ammoniak in Methanol und rührt 24 Stunden bei Raumtemperatur.

Nach Eindampfen im Vakuum hinterbleiben in einer Reinheit von 92 % (nach Gaschromatographie) 36,8 g (99 % der Theorie) (Z)-2-Methoximino-malonsäuremonoamid-ethylester als gelbbraunes Öl vom Brechungsindex $n_D^{20}$ = 1,4716.

Die Z-Konfiguration wurde durch $^{13}$C-NMR sichergestellt.

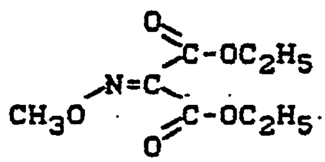

Zu einer Lösung von 108,9 g (0,5 Mol) 87prozentigem 2-Hydroximino-malonsäure-diethylester in 400 ml Dimethylsulfoxid gibt man 138 g (1 Mol) Kaliumcarbonat tropft in 20 Minuten 81,2 g (0,625 Mol) Dimethylsulfat zu, wobei die Temperatur auf 70 °C ansteigt, und rührt 3 Stunden bei 60 °C. Nach Abkühlung wird filtriert, das Filtrat in 1 l Wasser gegossen und dreimal mit je 600 ml Toluol/Essigester (5:1) extrahiert. Der mit 600 ml Wasser gewaschene Extrakt wird über Natriumsulfat getrocknet und im Vakuum eingedampft.

In einer Reinheit von 95 % (nach Gaschromatographie) erhält man so 73,9 g (69 % der Theorie) 2-Methoximinomalonsäure-diethylester als orangefarbenes Öl mit einem Brechungsindex $n_D^{20}$ = 1,4400.

Herstellung eines Folgeproduktes

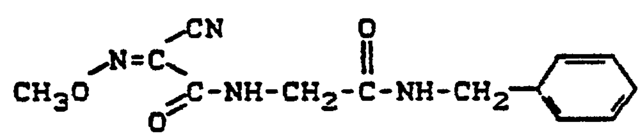

Zu einer Lösung von 12,3 g (0,05 Mol) Glycinbenzylamidhydrobromid in 100 ml trocknem Dimethylformamid gibt man 12,2 g (0,12 Mol) Triethylamin und bei 0 °C tropfenweise 7,4 g (0,05 Mol) (Z)-2-Cyan-2-methoximino-acetylchlorid und rührt das Reaktionsgemisch 1 Stunde bei 0° C und 17 Stunden bei Raumtemperatur. Das Lösungsmittel wird im Vakuum abdestilliert der Rückstand in 250 ml Dichlormethan gelöst, die Lösung mit 100 ml 1M Salzsäure, 100 ml gesättigter Natriumhydrogencarbonatlösung und 150 ml Wasser gewaschen, über Natriumsulfat getrocknet und im Vakuum eingedampft. Der Rückstand wird aus Toluol/Essigester/Ligroin (1:1:1) umkristallisiert.

Man erhält 7,35 g (53,6 % der Theorie) N$^{\alpha}$-[(Z)-2-Cyan-2-methoximino-acetyl]-glycinbenzylamid vom Schmelzpunkt 120-121 °C.

**Patentansprüche**

1. (Z)-2-Cyan-2-oximinoacetylchloride der allgemeinen Formel

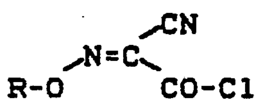

(I)

in welcher
R für gegebenenfalls ein- bis dreifach, gleich oder verschieden durch Halogen oder Methyl substituiertes Cycloalkyl mit 3 bis 6 Kohlenstoffatomen oder für die Gruppierung

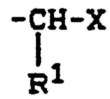

steht, wobei
$R^1$ für Wasserstoff oder Methyl steht und
X für Wasserstoff, für geradkettiges oder verzweigtes Alkyl mit 1 bis 8 Kohlenstoffatomen, für Vinyl, Ethinyl, Cyano, für gegebenenfalls ein- oder zweifach, gleich oder verschieden, durch Halogen substituiertes Cyclopropyl, für gegebenenfalls durch Halogen oder Alkyl mit 1 bis 4 Kohlenstoffatomen substituiertes Phenyl sowie für einen gegebenenfalls substituierten und gegebenenfalls mit einem Benzolring annellierten Heterocyclus steht, welcher 1 bis 3 gleiche oder verschiedene Heteroatome aus der Gruppe Stickstoffe, Sauerstoff und Schefel enthält und durch Halogen oder Alkyl mit 1 bis 4 Kohlenstoffatomen substituiert sein kann.

2. Verbindungen der allgemeinen Formel (I) gemäß Anspruch 1, worin
R für gegebenenfalls ein- oder zweifach, gleich oder verschieden durch Chlor oder Methyl substituiertes Cyclopentyl oder Cyclohexyl oder für die Gruppierung

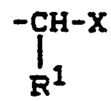

steht, wobei
$R^1$ für Wasserstoff oder Methyl steht und
X für Wasserstoff, Methyl, Ethyl, Venyl, Ethinyl, Cyano, für gegebenenfalls ein- oder zweifach durch Chlor substituiertes Cyclopropyl, für gegebenenfalls durch Chlor, Methyl oder Ethyl substituiertes Phenyl, für gegebenenfalls durch Chlor oder Methyl substituiertes Pyrazol-1-yl oder 1,2,4-Triazol-1-yl sowie für den Rest

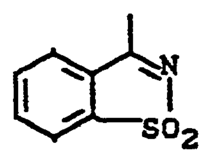

steht

3. Verfahren zur Herstellung von (Z)-2-Cyan-2-oximino-acetylchloriden der allgemeinen Formel (I)

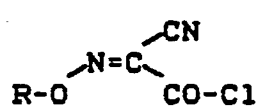

(I)

in welcher
R für gegebenenfalls ein- bis dreifach, gleich oder verschieden durch Halogen oder Methyl substituiertes Cycloalkyl mit 3 bis 6 Kohlenstoffatomen oder für die Gruppierung

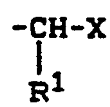

steht, wobei
$R^1$ für Wasserstoff oder Methyl steht und
X für Wasserstoff, für geradkettiges oder verzweigtes Alkyl mit 1 bis 8 Kohlenstoffatomen, für Vinyl, Ethinyl, Cyano, für gegebenenfalls ein- oder zweifach, gleich oder verschieden, durch Halogen substituiertes Cyclopropyl, für gegebenenfalls durch Halogen oder Alkyl mit 1 bis 4 Kohlenstoffatomen substituiertes Phenyl sowie für einen gegebenenfalls substituierten und gegebenenfalls mit einem Benzolring annellierten Heterocyclus steht, welcher 1 bis 3 gleiche oder verschiedene Heteroatome aus der Gruppe Stickstoff, Sauerstoff und Schwefel enthält und durch Halogen oder Alkyl mit 1 bis 4 Kohlenstoffatomen substituiert sein kann, dadurch gekennzeichnet, daß man Verbindungen der allgemeinen Formel (II)

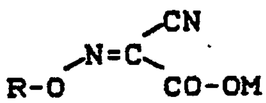 (II)

in welcher
R die oben angegebene Bedeutung hat und
M für Wasserstoff oder ein Alkalimetallatom wie beispielsweise Natrium oder Kalium steht,
mit einem Halogenierungsmittel gegebenenfalls in Gegenwart eines Verdünnungsmittels und gegebenenfalls in Gegenwart eines Katalysators umsetzt.

**Claims**

1. (Z)-2-Cyano-2-oximinoacetyl chlorides of the general formula

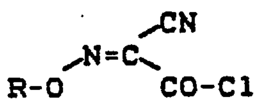 (I)

in which
R represents cycloalkyl, having 3 to 6 carbon atoms, which is optionally mono- or trisubstituted by halogen or methyl, the substituents being identical or different, or represents the

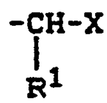

group,
where
$R^1$ represents hydrogen or methyl, and
X represents hydrogen, straight-chain or branched alkyl having 1 to 8 carbon atoms, vinyl, ethinyl, cyano, cyclopropyl which is optionally mono- or disbustituted by halogen, the substituents being identical or different, phenyl which is optionally substituted by halogen or alkyl having 1 to 4 carbon atoms, or an optionally substituted heterocyclic ring which is optionally fused to a benzene ring, contains 1 to 3 identical or different hetero atoms from the group comprising nitrogen, oxygen and sulphur, and may be substituted by halogen or alkyl, having 1 to 4 carbon atoms.

2. Compounds of the general formula (I) according to Claim 1, in which
R represents cyclopentyl or cyclohexyl which are optionally mono- or disubstituted by chlorine or methyl, the substituents being identical or different, or represents the

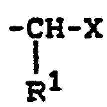

group,
where
$R^1$ represents hydrogen or methyl, and
X represents hydrogen, emthyl, ethyl, vinyl, ethinyl, cyano, cyclopropyl which is optionally mono- or disubstituted by chlorine, represents phenyl whcih is optionally substituted by chlorine, methyl or ethyl, represents pyrazol-1-yl or 1,2,4-triazol-1-yl, each of which is optionally substituted by chlorine or methyl, and represents the
radical.

3. Process for the preparation of (Z)-2-cyano-2-oximino-acetyl chlorides of the general formula (I)

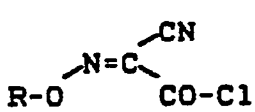 (I)

in which

R represents cycloalkyl, having 3 to 6 carbon atoms, which is optionally mono- or trisubstituted by halogen or methyl, the substituents being identical or different, or represents the

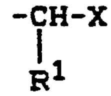

group,
where
$R^1$ represents hydrogen or methyl, and
X represents hydrogen, straight-chain or branched alkyl having 1 to 8 carbon atoms, vinyl, ethinyl, cyano, cyclopropyl which is optionally mono- or disbustituted by halogen, the substituents being identical or different, phenyl which is optionally substituted by halogen or alkyl having 1 to 4 carbon atoms, or an optionally substituted heterocyclic ring which is optionally fused to a benzene ring, contains 1 to 3 identical or different hetero atoms from the group comprising nitrogen, oxygen and sulphur, and may be substituted by halogen or alkyl, having 1 to 4 carbon atoms,
characterized in that compounds of the general formula (II)

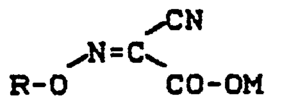 (II)

in which
R has the abovementioned meaning, and
M represents hydrogen or an alkali metal atom, such as, for example, sodium or potassium,
are reacted with a halogenating agent, if appropriate in the presence of a diluent and if appropriate in the presence of a catalyst.

**Revendications**

1. Chlorures de (Z)-2-cyano-2-oximino-acétyle suivant la formule générale

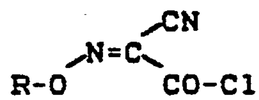 (I)

dans laquelle
R représente un radical cycloalkyle, éventuellement substitué une à trois fois, de manière identique ou différente, par un halogène ou un méthyle et comportant 3 à 6 atomes de carbone, ou bien le groupement

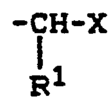

dans lequel
$R^1$ représente de l'hydrogène ou un radical méthyle, et
X représente de l'hydrogène, un alkyle à chaîne droite ou ramifiée à 1 à 8 atomes de carbone, un radical vinyle, éthynyle, cyano, un cyclopropyle éventuellement substitué une ou deux fois, de manière identique ou différente, par un halogène, un radical phényle substitué éventuellement par un halogène ou un alkyle à 1 à 4 atomes de carbone, ou un hétérocycle éventuellement substitué et accolé à un cycle benzénique, l'hétérocycle contenant de 1 à 3 hétéro-atomes identiques ou différents choisis dans le groupe de l'azote, de l'oxygène et du soufre et pouvant être substitué par un halogène ou un alkyle à 1 à 4 atomes de carbone.

2. Composés de la formule générale (I) suivant la revendication 1, dans laquelle
R représente un radical cyclopentyle ou cyclohexyle, éventuellement substitué une ou deux fois de manière identique ou différente par du chlore ou du méthyle, ou bien le groupement

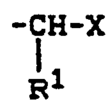

dans lequel
$R^1$ représente de l'hydrogène ou du méthyle, et
X représente de l'hydrogène, un radical méthyle, éthyle, vinyle, éthynyle, cyano, cyclopropyle éventuellement substitué une ou deux fois par du chlore, phényle éventuellement substitué par du chlore, du méthyle ou de l'éthyle, pyrazole-1-yle éventuellement substitué par du chlore ou du méthyle ou 1,2,4-triazol-1-yle, ou bien le radical

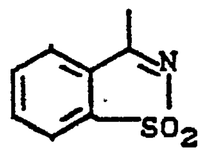

3. Procédé pour la préparation de chlorures de (Z)-2-cyano-2-oximino-acétyle suivant la formule générale (I)

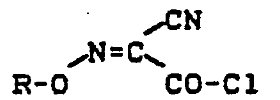

(I)

dans laquelle
R représente un radical cycloalkyle, éventuellement substitué une à trois fois de manière identique ou différente par un halogène ou un méthyle et 3 à 6 atomes de carbone, ou bien le groupement

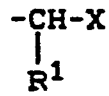

dans lequel
$R^1$ représente de l'hydrogène ou un radical méthyle, et
X représente de l'hydrogène, un radical alkyle à chaîne droite ou ramifiée à 1 à 8 atomes de carbone, un radical vinyle, éthynyle, cyano, un cyclopropyle éventuellement substitué une ou deux fois, de manière identique ou différente, par un halogène, un radical phényle substitué éventuellement par un halogène ou un alkyle à 1 à 4 atomes de carbone, ou un hétérocycle, éventuellement substitué et éventuellement accolé à un cycle benzénique, l'hétérocycle contenant de 1 à 3 hétéro-atomes identiques ou différents choisis dans le groupe de l'azote, de l'oxygène et du soufre et pouvant être substitué par un halogène ou un alkyle à 1 à 4 atomes de carbone,
caractérisé en ce que l'on fait réagir des composés de la formule générale (II)

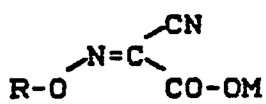

(II)

dans laquelle
R a la signification précitée, et
M représente de l'hydrogène, un atome de métal alcalin comme, par exemple, du sodium ou du potassium, avec un agent d'halogénation éventuellement en présence d'un diluant et, éventuellement, en présence d'un catalyseur.